(19) Europäisches Patentamt
European Patent Office
Office européen des brevets

(11) Numéro de publication: **0 177 414**
**A1**

(12) # DEMANDE DE BREVET EUROPEEN

(21) Numéro de dépôt: **85401878.5**

(22) Date de dépôt: **25.09.85**

(51) Int. Cl.[4]: **A 61 K 49/04**
C 07 C 103/44, C 07 C 103/49
//C07C103/28, C07C103/24

(30) Priorité: **09.10.84 FR 8415494**

(43) Date de publication de la demande:
**09.04.86 Bulletin 86/15**

(84) Etats contractants désignés:
**AT BE CH DE FR GB IT LI LU NL SE**

(71) Demandeur: **GUERBET S.A.**
**16-24 Rue Jean Chaptal**
**F-93601 Aulnay-sous-Bois Cedex(FR)**

(72) Inventeur: **Dugast-Zrihen, Maryse**
**107 rue Bobillot**
**F-75013 Paris(FR)**

(72) Inventeur: **Meyer, Dominique**
**23 rue du Taye**
**F-75013 Paris(FR)**

(72) Inventeur: **Guillemot, Michel**
**3 rue Lalo**
**F-75116 Paris(FR)**

(72) Inventeur: **Lautrou, Jean**
**21 avenue Gambetta**
**F-94160 Saint-Mande(FR)**

(72) Inventeur: **Bonnemain, Bruno**
**9 rue de Reims**
**F-77290 Mitry-Mory(FR)**

(74) Mandataire: **Bressand, Georges et al,**
**c/o CABINET LAVOIX 2 Place d'Estienne d'Orves**
**F-75441 Paris Cedex 09(FR)**

(54) **Composés triaminobenzéniques iodés, leur procédé de préparation et leur application dans des produits de contraste.**

(57) L'intention a pour objet des composés triaminobenzéniques iodés de type nonionique.

Ces composés sont utilisables dans des produits de contraste.

EP 0 177 414 A1

La présente invention concerne des composés utilisables dans des produits de contraste pour la radiographie.

On utilise depuis longtemps comme produits de contraste des composés iodobenzéniques présentant sur le noyau benzénique plusieurs atomes d'iode, en général 3 atomes d'iode par noyau benzénique, et divers autres substituants. Ces autres substituants sont des groupes pharmacologiquement acceptables qui permettent l'administration des composés chez l'homme et les animaux. Ces substituants sont d'une manière générale choisis, d'une part, pour conférer aux composés une hydrosolubilité suffisante en vue de l'administration de ces composés en solution aqueuse et, d'autre part, pour conférer à ces composés une tolérance suffisante par l'organisme humain.

A cet effet, on a proposé des structures non-ioniques, c'est-à-dire des dérivés iodobenzéniques possédant des substituants non-ioniques.

Ainsi, dans le brevet FR-A-2 053 037, on a proposé des composés carbamoyl iodobenzéniques contenant au total au moins un groupe N-hydroxy alcoyle et au moins deux groupes hydroxy.

Un composé illustrant cette classe est le métrizamide qui s'avère être toutefois d'une stabilité limitée.

La présente invention vise à fournir de nouveaux composés non-ioniques qui soient bien tolérés par l'organisme humain, très stables en solution aqueuse et qui puissent être obtenus aisément et avec un faible prix de revient.

A cet effet, la présente invention a pour objet des composés choisis parmi les composés de formule

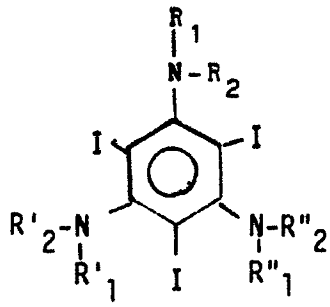

I

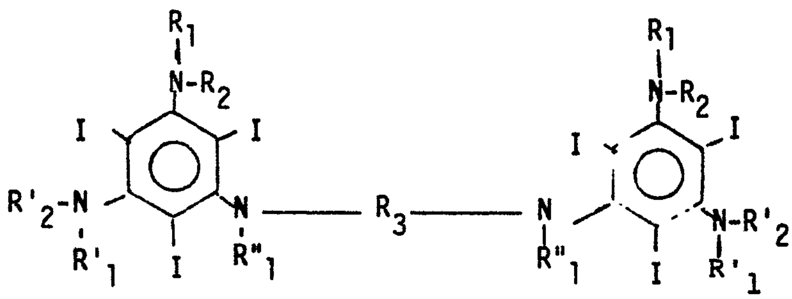

$II_a$

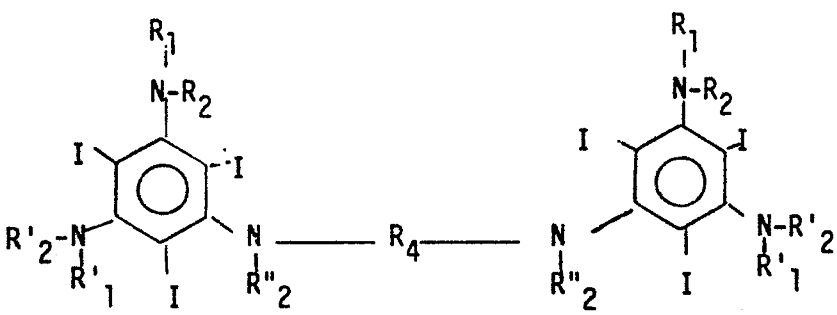

$II_b$

dans lesquelles

$R_1$, $R'_1$ et $R''_1$ sont choisis indépendamment les uns des autres parmi un atome d'hydrogène, un groupe alkyle en $C_1$ à $C_4$, un groupe hydroxyalkyle en $C_1$ à $C_4$, un groupe polyhydroxyalkyle en $C_1$ à $C_6$, un groupe alkoxy $(C_1-C_4)$ hydroxyalkyle $(C_1-C_4)$, et un groupe hydroxyalkoxy $(C_1-C_4)$ alkyle $(C_1-C_4)$,

$R_2$, $R'_2$ et $R''_2$ sont choisis indépendamment les uns des autres parmi les groupes acyle de formule

$$-\underset{\underset{O}{\|}}{C}-R_5$$

$R_5$ étant un groupe alkyle en $C_1$ à $C_4$, hydroxyalkyle en $C_1$ à $C_4$, polyhydroxyalkyle en $C_1$ à $C_6$ ou alkoxy $(C_1-C_4)$ alkyle $(C_1-C_4)$,

$R_3$ est un groupe choisi parmi les groupes diacyle de formule

$$-CO-R_6-CO-$$

$R_6$ étant une simple liaison, un groupe alkylène en $C_1$ à $C_4$, un groupe hydroxyalkylène en $C_1$ à $C_4$, un groupe polyhydroxyalkylène en $C_1$ à $C_4$ ou un groupe alkoxy $(C_1-C_4)$ alkylène $(C_1-C_4)$

$R_4$ est un groupe choisi parmi les groupes de formule

$$-CH_2-R_6-CH_2-$$

$R_6$ ayant la signification donnée précédemment.

Comme exemples de groupes $R_1$, $R'_1$ et $R''_1$ on peut citer $-H$, $-CH_3$, $-CH_2-CH_2-OH$, $-CH_2-CHOH-CH_2OH$, $-CH_2-C(CH_2OH)_3$, $-CH_2-CHOH-CH_2OCH_3$, $-CH_2-CH_2-O-CH_2-CH_2OH$, $-CH(CH_2OH)_2$.

Comme exemples de groupes $R_2$, $R'_2$ et $R''_2$ on peut citer $-CO-CH_3$, $-CO-CH_2OCH_3$, $-CO-CHOH-CH_3$, $-CO-CH_2OH$, $-CO-CH_2-CHOH-CH_2OH$, $-CO-CHOH-CH_2OH$, $-CO-CH-(CH_2OH)_2$, gluconyle, $-CO-C(CH_2OH)_2-CH_3$.

Pour des questions de facilité de synthèse une classe

préférée de composés de formule I est celle formée par les composés de formule

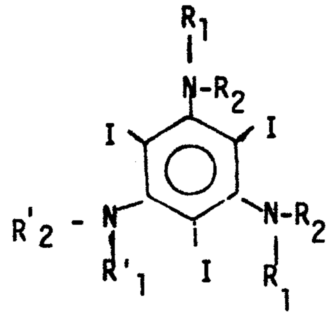

$I_a$

et plus particulièrement les composés symétriques de formule

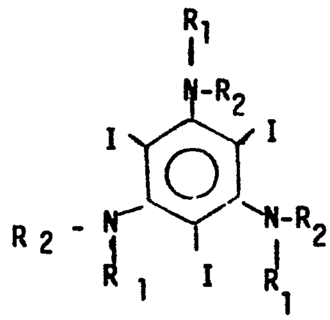

$I_{a'}$

$R_1$, $R'_1$, $R_2$ et $R'_2$ ayant les significations données précédemment.

Par ailleurs une autre classe préférée, en raison de leurs propriétés, est celle formée par les composés de formule I dans laquelle au moins l'un des substituants de chaque atome d'azote est un radical présentant au moins un groupe hydroxy ou alkoxy.

D'une manière générale les composés de formule I peuvent être préparés par acylation avec un réactif acylant $R_5$-CO-Z (Z étant un reste chlorure ou $-O-CO-R_5$) d'un composé choisi parmi les composés de formule

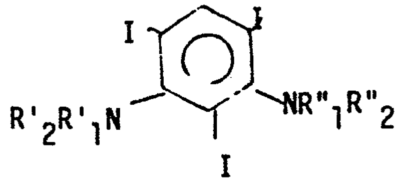

III

dans laquelle $R'_1$, $R''_1$, $R'_2$ et $R''_2$ ont les significations données précédemment ou sont des groupes correspondants dont les groupes hydroxy sont protégés,
et les composés de formule

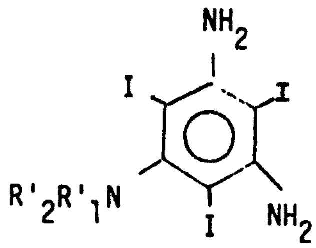

IV

dans laquelle $R'_1$ et $R'_2$ ont les significations données précédemment ou sont des groupes correspondants dont les groupes hydroxy sont protégés,
donnant respectivement des composés de formule

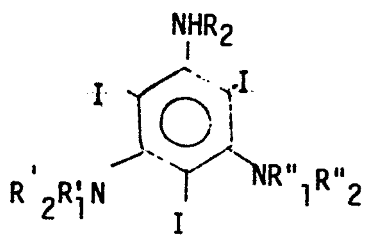

$I_b$

et

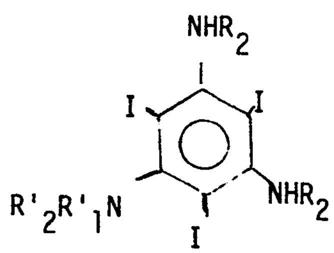

$I_c$

Cette réaction étant éventuellement suivie d'une déprotection des groupes hydroxy et/ou d'une alkylation par un agent alkylant $R_1$ Z' ($R_1$ ayant la signification donnée précédemment mais autre que l'hydrogène et Z'étant un groupe labile tel qu'un halogène) de façon à obtenir un composé de formule I ou Ia.

Comme agent acylant on utilise un chlorure d'acide ou un anhydride d'acide, les groupes hydroxy éventuels du groupe $R_2$ étant protégés. La réaction est effectuée en milieu anhydre éventuellement dans un solvant polaire, de préférence à une température de -10 à 100 °C, suivant les réactifs.

Comme agent alkylant on peut utiliser des dérivés chlorés, bromés, iodés. La réaction d'alkylation peut être effectuée dans un solvant aqueux ou organique, en présence d'une base notamment NaOH, KOH, $K_2CO_3$, NaH, MeONa. Eventuellement un groupe $R_1$ polyhydroxyalkyle peut être sous forme d'un précurseur (oxiranne ou groupe hydrocarboné insaturé tel qu'un groupe allyle que l'on oxyde ensuite en groupe polyhydroxy alkyle). La réaction peut également être effectuée en opérant dans des conditions de catalyse par transfert de phase.

Ces groupes hydroxy peuvent être protégés sous forme de groupe ester, acétal, éther. En cas de protection les groupes hydroxy sont déprotégés après la réaction d'alkylation.

Il est à noter que le procédé selon l'invention permet d'utiliser des vecteurs d'hydrophilie de faible coût tel que le chloropropane-2,3-diol ou l'épichlorhydrine. En outre dans les cas de composés symétriques (composés Ia'), ces vecteurs peuvent être introduits par trialkylation au dernier stade de synthèse, limitant ainsi les problèmes posés par la purification des composés hydrophiles.

Les composés aminés de formule III et IV peuvent être obtenus respectivement à partir des benzamides correspondants de formule

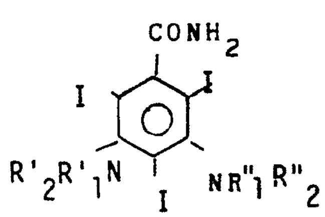

V

et

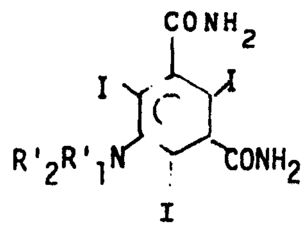

VI

par une réaction de réarrangement de type Hofmann.

Les réactifs préférés pour cette réaction sont les suivants : hypobromite de sodium, hypochlorite de sodium, méthylate de sodium-brome, tétracétate de plomb. L'encombrement stérique amené par les atomes d'iode en ortho et ortho' de la fonction benzamide limite le nombre des impuretés formées classiquement dans cette réaction, notamment les réactions parasites d'halogénation du cycle et les dimérisations des groupes isocyanate, ce qui permet d'obtenir des amines iodées avec de très bon rendement ( >90 %).

Les benzamides de formule V et VI peuvent être obtenus par des réactions classiques à partir de chlorures d'acides aromatiques triiodés, d'esters benzoiques ou isophtaliques non iodés, d'acides dinitrobenzoïque ou nitro isophtalique.

En particulier les benzamides de formule V peuvent être obtenus à partir d'un composé de formule

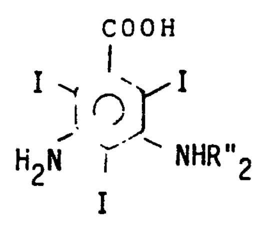

VII

par alkylation de façon à obtenir un composé de formule

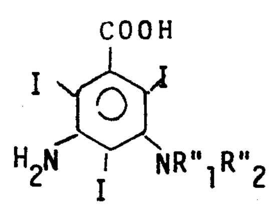

VIII

transformation de ce composé en chlorure d'acide

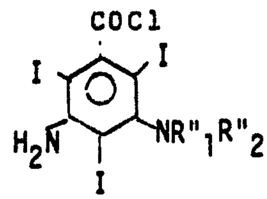

IX

conversion de ce composé en benzamide de formule

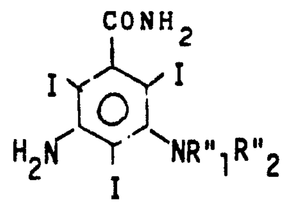

X

acylation de ce composé de façon à obtenir un benzamide de formule

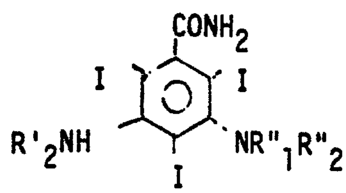

XI

et alcoylation de ce composé pour obtenir un benzamide de formule V.

Les benzamides de formule V présentant deux substituants identiques, à savoir les benzamides de formule

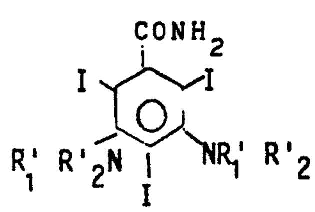

Va

peuvent être obtenus à partir d'un composé de formule

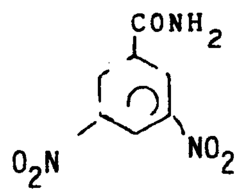

XII

par réduction en un composé de formule

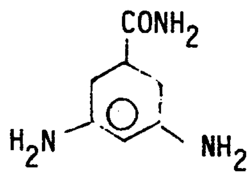

XIII

iodation du composé de formule XIII de façon à obtenir un composé de formule

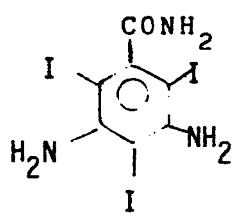

XIV

acylation de ce composé de façon à obtenir un composé de formule

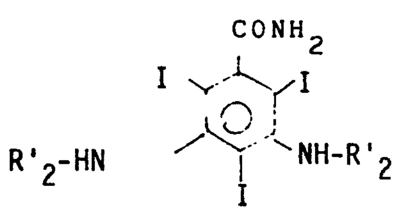

XV

et éventuellement alkylation du composé de formule VI en un composé de formule Va.

Les benzamides de formule VI peuvent être obtenus à partir de l'acide 5-nitroisophtalique par estérification en ester diméthylique, conversion de cet ester en 5-nitro-isophtalamide, réduction de ce composé en 5-aminoisophtal-amide et iodation du composé obtenu, acylation du composé

iodé obtenu de façon à obtenir un composé de formule

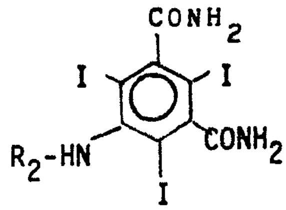

XVI

et éventuellement alkylation.

Les composés aminés de formule

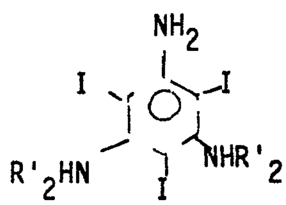

IIIa

peuvent en outre être obtenus à partir du 3,5-diamino-1-nitro-benzène par acylation en un composé de formule

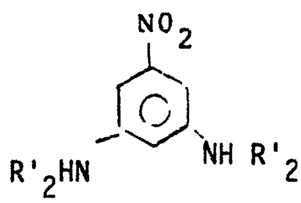

XVII

réduction en amine correspondante

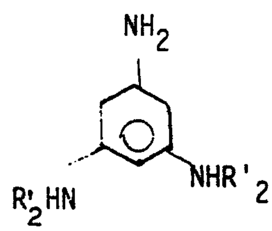

XVIII

et iodation.

Les composés aminés de formule IV peuvent de même être obtenus à partir de la 3,5 dinitroaniline par acylation en un composé de formule

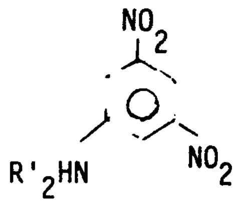

XIX

réduction en diamine de formule

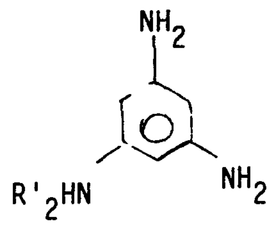

XX

iodation et éventuellement alkylation.

Les composés de formule $II_a$ peuvent être obtenus par réaction d'un composé de formule

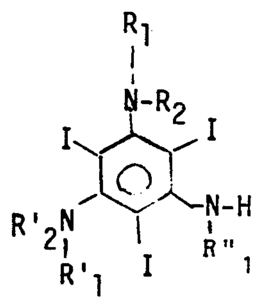

XXI

dans laquelle $R_1$, $R'_1$, $R''_1$, $R_2$ et $R'_2$ ont la signification donnée ci-dessus avec un agent biacylant de formule

$Cl - CO - R_6 - COCl$ XXII

Les composés de formule $II_b$ peuvent être obtenus par réaction d'un composé de formule I dans laquelle $R''_1$ est l'hydrogène avec un agent bialkylant de formule

$X - R_4 - X$ XXIII

dans laquelle X = Cl, Br ou I.

La présente invention a également pour objet des produits de contraste qui comprennent au moins un composé de formule I, $II_a$ et/ou $II_b$.

Ces produits de contraste sont utilisés chez l'homme et les animaux à des fins radiologiques.

La forme pharmaceutique préférée des produits de contraste selon l'invention est constituée par des solutions aqueuses des composés.

Les solutions aqueuses contiennent généralement un total de 5 à 100 g de composés pour 100 ml et la quantité injectable de telles solutions peut varier généralement de 1 à 1 000 ml.

Ces compositions peuvent être administrés par toutes les voies classiquement utilisées pour les produits de contraste non ioniques iodés et en particulier dans l'espace sous-arachnoidien et par voie parentérale (voie intraveineuse, intraartérielle, etc.).

On donnera ci-après un exemple de composition selon la présente invention

Composition

Composé de l'exemple 3 50 g

Eau pour préparation injectable

Q.S.P. 100 ml

Les exemples suivants illustrent la préparation des composés selon l'invention.

Dans ces exemples les composés ont été caractérisés par chromatographie en couche mince mesurée sur plaque de Silicagel 60 F 254 Merck avec les éluants suivants :

| | |
|---|---|
| Eluant 1 | Méthanol/Dichlorométhane (2/3) |
| Eluant 2 | Toluène/Méthyléthylcétone/Acide Formique (60/25/25) |
| Eluant 3 | Chloroforme/Méthanol/$NH_4OH$ (6/4/0,1) |
| Eluant 4 | Dioxanne/Eau/$NH_4OH$ (90/10/10) |
| Eluant 5 | Acétate d'éthyle/Isopropanol/$NH_4OH$ (55/35/20) |
| Eluant 6 | Dichlorométhane/Méthanol (90/10) |
| Eluant 7 | Dichlorométhane/Méthanol (80/20) |
| Eluant 8 | Butanol/Eau/Acide acétique (50/25/11) |

Dans ces exemples les spectres $^{1}H$ RMN ont été enregistrés sur un appareil Varian à 60 MHz.

Les déplacements chimiques exprimés en ppm sont mesurés par rapport au tétraméthyl silane utilisé comme référence interne.

EXEMPLE 1

Préparation du 2, 4, 6-triiodo-1, 3,5-triacétamidobenzène (première méthode)

a) Préparation du 3,5-dinitro-benzoate de méthyle

A une suspension de 214 g (1 mole) de l'acide 3,5-dinitrobenzoïque dans 930 $cm^3$ de méthanol anhydre sont ajoutés goutte à goutte 9 ml d'acide sulfurique concentré. Le mélange réactionnel est ensuite porté 15 heures à 70 °C, l'ester méthylique cristallisé est filtré puis lavé avec un minimum de méthanol (rendement 84 %).

F = 112 °C (littérature 113 °C)

CCM Eluant 1 Rf = 0,87

b) Préparation du 3,5-dinitrobenzamide

113 g (0,53 mole) de 3,5-dinitrobenzoate de méthyle sont dissous dans 225 ml de $CHCl_3$ auxquels sont ajoutés 565 ml d'ammoniaque à 28 %. Le système biphasique est agité une nuit à température ambiante. L'amide qui précipite du milieu réactionnel est filtré puis lavé à l'eau (rendement 82 %).

F = 182 °C (littérature 183 °C)

CCM Eluant 2 Rf = 0,6

c) Préparation du 3,5-diamino-2,4,6-triiodobenzamide

15 g (0,0284 mole) de 3,5-dinitrobenzamide et 0,1 g de charbon palladié à 5 % sont mis en suspension dans un autoclave avec 125 $cm^3$ de méthanol. La suspension est hydrogénée sous une pression de $3.10^5$ Pa et à une température de 70 °C. Le catalyseur est séparé par filtration, la solution résultante est introduite dans un mélange constitué de 125 $cm^3$ d'eau et 18 $cm^3$ d'acide chlorhydrique concentré. 46 $cm^3$ de chlorure d'iode (à 70 % en iode) sont ensuite introduits goutte à goutte à 60 °C. Après une nuit

à température ambiante, le précipité est filtré, lavé à l'eau, avec une solution bisulfitique, puis relavé à l'eau (rendement 91 %)

- Pureté en iode : 100,4%
- Point de fusion >300 °C avec décomposition
- CCM Eluant THF Rf = 0,92
  Eluant 2 Rf = 0,48

- Spectre RMN $^1H$ (DMSO-D6) 5,9,s ($NH_2$, 4H)
  2 s 7,3-7,5 (2H $CONH_2$) Disparition avec $D_2O$

d) Préparation du 3,5-di(N-acétylamino)-2,4,6-triiodobenzamide

41 g (0,0775 mole) de 3,5-diamino-2,4,6-triiodobenzamide sont introduits dans une solution à 80 °C de 100 cm³ d'acide acétique et 25 cm³ d'anhydride acétique. 2,2 cm³ d'acide sulfurique concentré sont ajoutés goutte à goutte et le mélange réactionnel est porté à 110 °C. Après 10 minutes de réaction, la suspension est refroidie à température ambiante puis reprise avec 100 cm³ d'eau. Le précipité obtenu est filtré puis redissous dans une solution de soude 2N. La solution est décolorée au noir et acidifiée avec HCl dilué.
Le 3,5 di(N acétylamino)-2,4,6-triodobenzamide précipite immédiatement. Après filtration le produit est lavé à l'eau et séché à 80 °C. Rendement 84 %.
- Pureté en iode 96 %
- CCM Eluant THF Rf = 0,56

e) Préparation de la 3,5 di(N-acétylamino)-2,4,6-triiodoaniline

20 g (0,0326 mole) de 3,5-di(N-acétylamino)-2,4,6-triiodobenzamide sont dissous dans une solution à 0 °C d'hypobromite de sodium préparée extemporanément (250 $cm^3$ d'eau, 6,5 g,de soude et 1,9 $cm^3$ de brome).
Le mélange réactionnel est agité ½ heure à température ambiante, puis chauffé 5 heures à 70 °C.
Après une nuit à température ambiante le précipité formé est filtré puis lavé à l'eau. Rendement 90 %
- Pureté en iode 99 %
- CCM Eluant THF Rf = 0,77
- Spectre infrarouge conforme.

f) Préparation du 2,4,6-triiodo-1,3,5-triacétamidobenzène

31 g (0,053 mole) de 3,5 di(N-acétylamino)-2,4,6-triiodoaniline sont ajoutés à une solution de 31 $cm^3$ d'acide acétique et 10 $cm^3$ d'anhydride acétique à 80 °C. Après addition goutte à goutte de 2 $cm^3$ d'acide sulfurique concentré, la solution est laissée à 105 °C quelques minutes. Le milieu réactionnel est refroidi à la température ambiante puis repris à l'eau. Le précipité obtenu est filtré, lavé à l'eau et séché. Rendement 97 %.
- Pureté en iode 98 %
- Spectre infrarouge et Spectre RMN conformes
- CCM Eluant 3 Rf = 0,84

EXEMPLE 2

Préparation du 2,4,6-triiodo-1,3,5-triacétamidobenzène

a) Préparation du 5-nitro-isophtalate de méthyle

1,5 mole (320 g) d'acide 5-nitro-isophtalique sont dissous dans 1,4 l de méthanol et 13 ml d'acide sulfurique concentré.

Le mélange réactionnel est chauffé 10 heures en reflux.

Après cristallisation progressive de l'ester, le produit est filtré puis séché à l'étuve à 70 °C.
- F = 123 °C
- CCM Eluant 4 Rf = 0,9
  Eluant 5 Rf = 0,9

b) Préparation du 5-nitro-isophtalamide

25 g de 5-nitro-isophtalate de méthyle (0,105 mole) en suspension dans 250 ml de méthanol et 80 ml d'ammoniaque 10 N sont chauffés 16 heures à 40 °C. Le produit obtenu est essoré et lavé au méthanol.

- Rendement 73 %
- CCM Eluant 4 Rf=0,8
- F = 225 °C

c) Préparation du 2,4,6-triiodo-5-amino-isophtalamide

10 g de 5-nitro-isophtalamide (0,048 mole) sont mis en suspension dans 400 cm³ d'eau et 50 cm³ d'acide chlorhydrique 5N, puis hydrogénés 1 heure à température ambiante sous une pression de $3.10^5$ Pa en présence de noir palladié. Après filtration du catalyseur les jus d'hydrogénation sont iodés directement par addition goutte à goutte à 70 °C de 34,8 cm³ de chlorure d'iode (70 % en $I_2$). Le milieu réactionnel est laissé 48 heures à 70 °C sous agitation. Après refroidissement à température ambiante, l'amine triodée est essorée, lavée au bisulfite dilué à l'eau puis séché à l'étuve à 80 °C.

- Rendement 85 %
- CCM Eluant 5 Rf = 0,5
  Eluant 2 Rf = 0,45
- pureté en iode 98 %
- spectre infrarouge conforme

d) Préparation du 2,4,6-triiodo-5-acétamido isophtalamide

16 cm³ de chlorure d'acétyle (0,224 mole) sont ajoutés goutte à goutte à une suspension constituée de 64 g de 2,4,6-triiodo-5-amino isophtalamide dans 120 cm³ de diméthylacétamide anhydre.

Au cours de l'addition la température s'élève jusqu'à 35 °C. Après dissolution complète du produit de départ, le produit acylé précipite progressivement. Le précipité est essoré, lavé à l'eau et à l'acétone, puis séché 16 heures à l'étuve (70 °C).

- Rendement 89 %
- pureté en iode 98,5 %
- CCM Eluant 2 Rf = 0,3
  Eluant $CHCl_3/CH_2OH$ (60/40) Rf= 0,65
- spectre infrarouge conforme.

e) <u>Préparation du 2,4,6-triiodo-5-acétamido-1,3-diamino benzène</u>

14 g de 2,4,6-triiodo-5-acétamido isophtalamide (0,023 mole) sont ajoutés à une solution à 0 °C d'hypobromite de sodium préparée extemporanément par addition de 2,36 $cm^3$ de brome dans 460 $cm^3$ de soude 1N.

Le mélange réactionnel est agité ½ heure à température ambiante puis chauffé 5 heures à 70 °C, le produit réactionnel précipite lentement. Après refroidissement à température ambiante, le produit est essoré, lavé à l'eau et séché à l'étuve à 70 °C

- Rendement 59,2 %

Un deuxième jet est obtenu par précipitation à l'acide chlorhydrique

- Rendement global 81 %
- pureté en iode 99,7 %
- dosage d'acidité au méthylate de sodium 100,1 %
- CCM Eluant 2 Rf = 0,65
- Spectre infrarouge conforme

f) <u>Préparation du 2,4,6-triiodo-1,3,5-tri-acétamidobenzène</u>

A une suspension de 7,1 g de 2,4,6-triiodo-5-acétamido 1,3-diaminobenzène dans 14 $cm^3$ de DMAC sont ajoutés goutte à goutte 3,8 $cm^3$ de $CH_3COCl$.

Après 16 heures de réaction à température ambiante, le produit qui a précipité dans le milieu réactionnel est essoré, lavé à l'éther puis à l'eau afin d'éliminer le DMAC et l'acide acétique formé.

- rendement 83 %
- pureté en iode 97,6 %
- CCM Eluant 2 Rf = 0,25
  Eluant 3 Rf = 0,84

<u>EXEMPLE 3</u>

<u>Préparation du 2,4,6-triiodo-1,3,5-tris [N-(2,3-dihydroxy propyl)acétamido]benzène (Ière Méthode)</u>

10 g de 2,4,6 triiodo-1,3,5-tri acétamido benzène (0,016 mole) sont dissous dans une solution à 40 °C cons-

tituée de 100 $cm^3$ d'eau et 4 g de soude en pastilles. 11 $cm^3$ de chloro-propane-2,3-diol sont additionnés goutte à goutte au milieu réactionnel. Après 5 heures de réaction à 70 °C sous agitation, la suspension obtenue est concentrée à sec puis reprise avec de l'alcool isopropylique. Le précipité séparé par filtration est purifié après dissolution dans l'eau par déminéralisation par passages successifs sur résines $H^+$ et $OH^-$ (Amberlite 77 et 78). L'évaporation à sec de la solution permet de récupérer un produit blanc avec un rendement après séchage de 52 %

- Pureté en iode : 97 %

- CCM Eluant 3 Rf = 0,56 et 0,38 (présence de 2 isomères)

Spectrométrie de masse (FAB).
Pic de masse 850 $[MH^+]$

- Spectre RMN $^1H$ ( DMSO D6 + $D_2O$)

2s 1,90,2,4 ($COCH_3$ 9H)
m 3,5 ($CH_2$-CH-$CH_2$. 15H)

$^{13}C$ RMN (DMSO) (déplacement chimique en ppm par rapport au tétraméthylsilane)

22,4 ($\underline{CH_3}$-$\underset{\|}{C}$) ; 52,66 ($\underline{CH_2}$-N) ;
O

64,3 ($CH_2$-O) ; 69,7 (CH) ;

108,9 - 110,5 - 111,8 (C-I) ;

$\underline{C}$ - N (150,25) ; $\underline{C}$ = O (169,25)

## EXEMPLE 4

Préparation du 2,4,6-triiodo-1,3,5 tris [N-(2,3-dihydroxypropyl)acétamido]benzène (2ème méthode avec catalyse par transfert de phase)

On dissout 12 g du 2,4,6 triiodo-1,3,5-triacétamido benzène

dans un système biphasique composé de 400 $cm^3$ de toluène et 72 $cm^3$ d'eau contenant 14 g de soude en pastille.

19,6 g de tétrabutylammonium hydrogénosulfate, et 25 $cm^3$ de 2,2-diméthyl-4-bromométhyl-1,3-dioxolanne sont additionnés sous agitation violente. Après 8 heures de réaction à 80 °C, l'interphase formée et la phase toluénique sont décantées, reprises à l'acétate d'éthyle et lavées abondamment à l'eau; après séchage de la phase organique sur $MgSO_4$ , évaporation à sec et reprise par un mélange éther isopropylique-éther de pétrole, on récupère 7,7 g de produit cristallisé.

- Rendement 41,5 %
- Pureté en iode 99 %
- CCM Eluant 6 $R_f$ = 0,76-0,70-0,56
- Spectre RMN $^1H$ ($CDCl_3$)
  d 1,1-1,4 ($CH_3$-C-$CH_3$ 18 H)
  2 s. 1,9-2,3 ($COCH_3$ 9H)
  Massif complexe 3 à 4,8
  ($CH_2$-CH-$CH_2$ 15H)

Ce produit est ensuite déprotégé par dissolution dans 80 $cm^3$ d'éthanol en présence de 5 g de résine $H^+$ Amberlite 15. Après 3 jours de réaction la résine est filtrée et la solution concentrée à sec, le produit huileux est repris à l'éther puis filtré.

- CCM Eluant 3 $R_f$=0,56 et 0,38 2 isomères
- Rendement 75 %
- Pureté en iode 100,9 %

EXEMPLE 5

Préparation du 2,4,6-triiodo-1,3,5-tris[N-(1,3-dihydroxypropyl) acétamido]benzène.

2 g de 2,4,6-triiodo-1,3,5-triacétamido benzène (3,2 mmoles)

sont dissous dans 20 $cm^3$ d'eau et 3,5 $cm^3$ de soude 5N à 60 °C. 1,4 $cm^3$ de chloropropane-1,3-diol sont ensuite additionnés goutte à goutte en maintenant la température à 60 °C pendant 6 heures.

Le milieu réactionnel est évaporé partiellement puis laissé 4 jours à 0 °C.

Après filtration, le produit brut récupéré est chromatographié sur silice silanisée (Merck 60) par élution successive à l'eau et au méthanol.

Rendement 22 %

Pureté en iode = 98,8 %

- CCM Eluant 3 présence de 2 isomères $R_f$ 0,55 et 0,38

EXEMPLE 6

2,4,6-triiodo-1,3,5-tris[ N-(2-(2-hydroxyéthoxy)éthyl) acétamido]benzène

10 g de 2,4,6 triiodo-1,3,5-triacétamido benzène sont ajoutés sous atmosphère anhydre à une suspension de 2,46 g d'hydrure de sodium dans 200 $cm^3$ de DMAC sec.

Après une heure de réaction, 30 g de 2-(2'-chloroéthoxy) éthanol protégé sous forme d'éther tétrahydropyrannyle sont ajoutés goutte à goutte.

Le milieu réactionnel est maintenu 12 heures à 60 °C puis évaporé à sec.

L'huile résultante est purifiée sur colonne de silice (éluant $CH_2Cl_2$/MeOH 95/5). $R_f$=0,62, 0,70, 0,77.

Le produit huileux récupéré (19,6 g) est déprotégé par catalyse sur résine Amberlyst 15 dans le méthanol. Après évaporation à sec, le produit est chromatographié sur silice (éluant $CH_2Cl_2$/MeOH 90/10)

- Rendement 40 %
- Pureté en iode 99 %
- CCM Eluant 6 3isomères $R_f$ 0,25 - 0,35 - 0,4

- Spectre RMN $^1$H ($DMSOD_6 + D_2O$)
  2 s, 1,8 et 2,2 (9H $COCH_3$);
  s, 3,4 ($CH_2-O-CH_2$ 12H);
  s, 3,7 ($-N-CH_2$, $-CH_2-O$, 12H)

EXEMPLE 7

2,4,6-triiodo-1,3,5-tris [N-(2-hydroxyéthyl)acétamido] benzène

6,4 $cm^3$ de 2-chloro-éthanol sont ajoutés goutte à goutte sur une solution composée de 10g (0,0159 mole) de 2,4,6 triiodo-1,3,5 triacétamido benzène dans 120 $cm^3$ d'eau et 20 $cm^3$ de soude 5N.
Après 24 heures de réaction à 70 °C, le précipité formé est filtré, lavé à l'eau glacée, puis séché une nuit à l'étuve à 60 °C.
- Rendement 60 %
- Pureté en iode 99 %

CCM Eluant 7 $R_f$ = 0,49-0,52
Eluant 2 $R_f$ = 0,12

- Spectre RMN $^1$H (DMSO D6 + $D_2O$)
  2s, 1,9 et 2,4 (9H $COCH_3$);
  s, mal résolu 3,7 ($CH_2CH_2$, 12 H)

EXEMPLE 8

a) Préparation du 2,4,6-triiodo-3,5-bis-[N-(2,3-dihydroxy propyl)acétamido]-aniline.

Dans une solution composée de 75 g de 2,4,6 triiodo-3,5-bis (acétamido)-aniline dans 910 $cm^3$ d'eau et 300 $cm^3$ de soude 10N, 130 $cm^3$ de chloropropane-2,3-diol sont ajoutés goutte à goutte à 70 °C.
Après 12 heures de réaction à 70 °C le milieu réactionnel est refroidi, neutralisé, par addition d'acide chlorhydrique

concentré puis décoloré au noir, 223 g d'huile sont récupérés après évaporation à sec de la solution, reprise à l'éthanol, filtration des sels et concentration à sec. Cette huile est purifiée par chromatographie sur silice silanisée Merck par élution à l'eau puis avec un gradient eau/Méthanol 9/1 à 5/5

- Rendement 72 %
- CCM Eluant 7 2 isomères $R_f$ = 0,45 et 0,51
- Pureté en iode 102,2 %

b) Préparation du 2,4,6-triiodo-1(méthoxyacétamido-3,5-bis [N-(2,3-dihydroxypropyl)acétamido]benzène

60 $cm^3$ du chlorure de l'acide méthoxyacétique sont ajoutés goutte à goutte sous atmosphère anhydre sur une solution de 40 g du produit décrit en a) dans 350 $cm^3$ de DMAC anhydre.

Le mélange réactionnel est laissé 3 heures à 60 °C puis concentré à sec.

Le résidu est repris par du dichlorométhane. La phase organique est lavée plusieurs fois à l'eau, puis évaporée à sec, l'huile récupérée est dissoute dans 500 $cm^3$ de méthanol et 50 $cm^3$ de soude 5N.

La solution est laissée une nuit à température ambiante puis évaporée à sec.

Le produit obtenu est purifié par chromatographie sur silice silanisée (éluant $H_2O$) et déminéralisation sur résine $H^+$ et $OH^-$.

- Rendement 55 %
- Dosage d'acide au méthylate de sodium 105 %
- Pureté en iode 99,2 %
- CCM Eluant 7 2 isomères $R_f$ = 0,59 et 0,42
- Spectre RMN $^1H$ (DMSO + $D_2O$)

2s 1,75, 2,3 ($COCH_3$,9H);
m 3,2 à 3,6 ($CH_2$-CH-$CH_2$ 10H)
s 3,5 ($OCH_3$ 3H); s 4 ($\underline{CH_2}$-$OCH_3$2H)

EXEMPLE 9

Préparation du 2,4,6-triiodo-1-(2-hydroxypropionyl-amino)-3,5-bis [N-(2,3-dihydroxy propyl)acétamido]benzène

100 g du chlorure de l'acide lactique O-acétylé sont additionnés lentement à une solution de 45 g de 2,4,6-triiodo-3,5-bis [N-(2,3 dihydroxypropyl)acétamido]aniline dans 400 $cm^3$ de DMAC anhydre. Après 16 heures de réaction à 50 °C, la solution est concentrée à sec, puis reprise par du dichlorométhane. La phase organique est lavée 3 fois à l'eau bicarbonatée, séchée sur sulfate de magnésium, décolorée au noir et concentrée à sec.

Le résidu huileux est repris par 500 $cm^3$ de MeOH et 60 $cm^3$ de soude 5N pendant 12 heures à température ambiante.

L'évaporation à sec de cette solution permet de récupérer une huile qui est purifiée par chromatographie sur silice (gradient $CH_2Cl_2$/MeOH 8/2) et déminéralisée sur résine $H^+$ et $OH^-$

- Rendement 21%
- Pureté en iode 100,2 %

CCM Eluant 7 2 isomères $R_f$ = 0,31 et 0, 20

Spectre RMN $^1H$ (DMSO D6 + $D_2O$)

d 1,4 ($\underline{CH_3}$-C, 3H) ; 2 s

1,75, 2,2 ($COCH_3$, 9H) ;

m 3,3 ($CH_2$-CH-$CH_2$ 11H)

EXEMPLE 10

Préparation du 2,4,6-triiodo-1,3,5-tris(méthoxyacétamido) benzène

a) Préparation du 1-nitro-3,5-bis[méthoxyacétamido]benzène

Une solution de 0,468 mole du chlorure de l'acide méthoxy acétique dans 110 $cm^3$ de DMAC anhydre est ajoutée goutte à goutte à 0 °C sur une solution composée de 30 g (0,195 mole) de 3,5-diamino-nitro benzène (G.H.DENNY et Coll. J. Med

1976 19 (3)426) dissous dans 80 cm³ de DMAC anhydre et 35 cm³ de pyridine.

Le milieu réactionnel est maintenu 2 heures à température ambiante, puis filtré.

Le précipité est lavé à l'eau, puis à l'éthanol. Après séchage à 40 °C sous vide, on récupère 55 g de produit.

- Rendement 95 %
- CCM Eluant 2 $R_f$ = 0,47

b) Préparation du 2,4,6-triiodo-3,5-bis[méthoxy acétamido] aniline

20 g du composé obtenu en a) sont réduits dans 100 cm³ d'acide acétique à 80 °C en présence de palladium sur charbon sous une pression de $6.10^5$ Pa d'hydrogène.

Après filtration du catalyseur, la solution acétique est diluée par 500 cm3 d'eau auxquels on ajoute 18 cm3 d'acide chlorhydrique concentré, puis 39,5 cm³ de chlorure d'iode à 70 %.

Le précipité est essoré après 5 heures de réaction, lavé à l'eau bisulfitée puis séché à l'étuve sous vide 16h à 70 °C.

- Rendement 80 %
- CCM Eluant 2 $R_f$ = 0,5
  Eluant 8 $R_f$ = 0,70
- Pureté en iode 98,8 %

c) Préparation du 2,4,6-triiodo-1,3,5 tris [méthoxy acétamido]benzène

0,054 moles de chlorure de l'acide méthoxy acétique en solution dans 100 cm³ de DMAC sec sont ajoutés lentement sur une solution de 23 g (0,036 mole) du 2,4,6-triiodo-3,5-bis [méthoxy acétamido]aniline dissous dans 125 cm³ de DMAC anhydre, la réaction est laissée 1 heure à 0 °C puis traitée par addition d'eau glacée. Après essorage, lavage à l'eau et séchage à l'étuve sous vide à 40 °C, le produit est récupéré.

- Rendement de 70 %
- CCM Eluant 2 $R_f$ = 0,35

Eluant 8 $R_f$ = 0,70

- Pureté en iode = 98,8 %
- Spectre RMN $^1H$ (DMSO D6)

s 3,5 ($OCH_3$, 9H)

s 4,05 ($CO\underline{CH_2}$-O, 6H)

3s 8,45, 9,3, 10 (NHCO,3H)

Disparition avec $D_2O$

EXEMPLE 11

Préparation du 2,4,6-triiodo-1,3,5-tris-[ N-(2,3-dihydroxy-propyl)méthoxy acétamido]benzène

2 g (2,8.$10^{-3}$ moles) de 2,4,6-triiodo-1,3,5-tris (méthoxy amido)benzène sont dissous dans 24 cm³ d'eau et 1 g de soude à 70 °C

0,01 mole de chloropropane-2,3-diol sont ajoutés lentement et la réaction est maintenue 2 h 30 à 70 °C. Le milieu réactionnel est évaporé à sec puis repris dans l'éthanol absolu. Les sels minéraux sont éliminés par filtration. Après évaporation à sec de la solution, le produit est purifié par chromatographie sur silice silanisée (éluant : eau-méthanol)

- Rendement 55 %
- CCM Eluant 2 $R_f$ = 0,67

Eluant 6 2 isomères $R_f$ = 0,20 et 0,47

- Pureté en iode = 99 %

EXEMPLE 12

Préparation du 2,4,6-triiodo-1,3,5-tris[ N-(2-hydroxyéthyl) méthoxy acétamido]benzène

2 g (2,8.$10^{-3}$ mole) de 2,4,6-triiodo-1,3,5-tris[méthoxy acétamido]benzène sont dissous dans 24 $cm^3$ d'eau et 1 g de soude. 0,01 mole de 2-chloro éthanol est ajoutée goutte à goutte. Après 2 heures de chauffage à 70 °C, on constate l'apparition d'une gomme, celle-ci est filtrée puis reprise à l'éthanol absolu afin d'éliminer les sels minéraux. Le produit est ensuite purifié sur silice silanisée (éluant eau-méthanol).

- Rendement 50 %
- Pureté en iode : 99,6 %
- CCM Eluant 2 2 isomères $R_f$ = 0,18, 0,25

  Eluant 6 4 isomères $R_f$ = 0,25-0,27

  0,36-0,40

- Spectre RMN $^1H$ ( DMSO D6)

  s. 3,3 ($OCH_3$, 9H)

  m. 3,5 à 3,9 ($-\underset{\underset{O}{\|}}{C}-CH_2-O$,

  $CH_2CH_2$ 18H)

EXEMPLE 13

Préparation du 2,4,6-triiodo-1-méthoxyacétamido-3,5-bis (2-hydroxypropionamido)benzène.

a) Préparation du 1-nitro-3,5-bis(2-acétoxy propionamido) benzène

Une solution de 0,23 mole de chlorure d'acide L.lactique O acétylé dans 100 ml de DMAC anhydre est ajoutée goutte à goutte à 0 °C sur une solution contenant 15,3 g (0,1 mole) de 3,5-diaminonitrobenzène dissous dans 50 ml de DMAC et 30 ml de triéthylamine. La température est maintenue vers 10 °C durant 1 h 30 avant de laisser remonter le milieu à température ambiante Le chlorhydrate de $Et_3N$ est filtré et la solution est versée sur de l'eau. Après essorage, le produit est recristallisé dans un mélange acétate d'éthyle/éther de pétrole (4/1) avec un rendement global de 60 %

- CCM (Eluant 2) $R_f$ = 0,60
- RMN($CDCl_3$): 1,5-1,6 (d 6H) ; 2,2 (s 6H) ; 5,1 (q 2H) 8,1 (m 2H) ; 9 (m 1H)

b) Préparation du 2,4,6-triiodo-3,5-bis-(2-acétoxy propionylamino)aniline

7,6 g ($2.10^{-2}$ mole) de 1-nitro-3,5-bis(2-acétoxy propionamido)benzène sont réduits dans 75 ml de méthanol à température ambiante en présence de Pd/C sous une pression de $4.10^5$ Pa d'$H_2$ durant 5 heures. Après filtration du catalyseur la solution de réduction est diluée par 250 ml d'$H_2O$, acidifiée par 3 ml d'HCl puis chauffée à 65 °C. L'iodation s'effectue par addition de 12 ml d'une solution de chlorure d'iode à 70 %. Le dérivé iodé précipite. Après essorage et claırçage à l'eau on récupère 3g de composé

- Rendement 20 %
- CCM Eluant 2 $R_f$ = 0,65
- Dosage d'iode pureté 99,5 %
- RMN (DMSO) 1,4-1,5 (d 6H) ; 2,05 (s 6H) 4 (massif large 2H); 5,2 (q 2H); 10(m 2H)

c) Préparation du 2,4,6-triiodo-1-(méthoxyacétyl amino)-3,5-bis-(2-acétoxy propionamido)benzène

A une solution de 0,005 mole de chlorure d'acide méthoxy acétique dans 5 ml d'un mélange de DMAC/dioxanne (3/2) on ajoute à 2-5 °C 2,2 g soit 0,003 mole de 2,4,6-triiodo-3,5-bis-(2-acétoxy propionamido)aniline.
Après 24 h d'agitation à température ambiante la solution est évaporée et l'huile est cristallisée dans l'éther éthylique et l'acétone.

- Rendement : 33 %
- Dosage d'iode pureté 99,5 %
- CCM (THF 80/$CHCl_3$20) $R_f$ = 0,85
- RMN (DMSO + $CDCl_3$) 1,6 (d, 6H) ; 2,2 (s 6H) ; 3,6 (s 3H) ; 4,1 (s 2H) ; 5,4 (m 2H) ; 9,3-9,8 (m 3H).

d) Préparation du 2,4,6-triiodo-1-méthoxyacétamido-3,5-bis-(2-hydroxy propion amido)benzène.

19 g de 2,4,6-triiodo-1-(méthoxyacétamido)-3,5-bis-(2-acétoxy propionamido) benzène sont dissous dans 125 ml de méthanol en présence de 10 ml de soude 1N.
Après 3h à température ambiante la réaction est complète. La solution est filtrée, évaporée et le produit cristallise

dans le dioxanne après déminéralisation sur résine échangeuse d'ions.

- Rendement 25%
- CCM éluant 2 $R_f$ = 0,30
- Dosage d'iode pureté 96%
- RMN 1H (DMSO) 1,4-1,5 (d 6H); 3,5 (s,3H); 4,1 (m,4H); 5,5 (m, 2H); 9,7-10 (m, 3H)

EXEMPLE 14

Préparation du 2,4,6-triiodo-1-(2-hydroxy-propionamido) 3,5-bis(méthoxy acétamido)benzène

a) préparation du 2,4,6-triiodo-1-(2-acétoxy propionamido) 3,5-bis (méthoxy acétamido)benzène

Une solution fraichement préparée de 0,03 moles de chlorure de l'acide L-lactique O-acétylé dans 10 cm³ de DMAC est ajoutée goutte à goutte à 0 °C sur une solution contenant 6,5 g (0,01 mole) de 2,4,6-triiodo-3,5-bis-(méthoxy acétamido)aniline dissous dans 10 cm³ de DMAC. Un effet thermique se développe lors de l'addition puis on agite pendant 12 h en laissant revenir le milieu à température ambiante. Après dilution du milieu par addition d'un volume d'acétone et de trois volumes d'éther, le produit cristallise.

- Rendement 71 % (5g)
- Dosage d'iode pureté 95 %
- CMM éluant : Tétrahydrofuranne/$CHCl_3$ (80/20) $R_f$= 0,70
- RMN (DMSO) 1,5-1,6 (d, 3H) ; 2,2 (s, 3 H) ;3,5 (s,6 H) ; 4,1(s,4 H) ; 5,3 (m,1 H); 10,1-10,3 (m 3 H).

b) Préparation du 2,4,6-triiodo-1-(2-hydroxy propionamido) 3,5-bis-(méthoxy acétamido)benzène.

5,5g de 2,4,6-triiodo-1-(2-acétoxy propionamido)-3,5-bis-(méthoxy acétamido)benzène sont dissous dans 25 ml de méthanol en présence de 0,5g de $K_2CO_3$. Après agitation pendant 3 h à température ambiante, le milieu est filtré, et le filtrat évaporé. L'huile obtenue est cristallisée dans le dioxanne.

- Rendement 29%
- CCM (éluant 2) Rf = 0,35
- RMN (DMSO)1,4-1,5 (d, 3H); 3,5 (s, 6H); 4 (m, 5H); 5,6 (m, 1H); 9,9 (m, 3H)

EXEMPLE 15

Préparation du 2,4,6-triiodo-1-acétamido- 3,5-[N-(2,3-dihydroxypropyl) acétamido] benzène

4,5 g de 2,4,6-triiodo-3,5-bis [N-(2,3-dihydroxypropyl) acétamido] aniline sont ajoutés sur une solution composée de 5,85 $cm^3$ d'anhydride acétique et 50 $cm^3$ d'acide acétique anhydre à 80°C. La réaction est catalysée par addition de 0,5 $cm^3$ d'acide sulfurique concentré, puis maintenue à 80°C pendant 1/2 heure. Le mélange réactionnel est refroidi, hydrolysé par 20 $cm^3$ d'eau, puis concentré à sec.

L'ester formé est saponifié directement par addition de 40 $cm^3$ de méthanol et 5 $cm^3$ de soude 10 N.

Après concentration à sec, le produit est repris par l'eau et purifié par chromatographie sur silice silanisée (élution $H_2O$).

Rendement : 67%

- CCM éluant 7   Rf = 0,32
- RMN $^1H$ ($DMSOD_6$) : 3 s, 1,8 -2,1 -2,2 (9H, $COCH_3$) ; m 3,4 - 3,9 (10 H, $CH_2$ - CH); s, 4,5 (4H, OH); s, 10,1 (1H, NH). Disparition avec $D_2O$ des pics à 4,5 et 10,1 ppm.

EXEMPLE 16

Préparation du 1,3-bis-{N-(2,4,6-triiodo-3,5-bis[ N-(2,3-dihydroxypropyl)acétamido]phényl)acétamido}propane

a) préparation du 1,3-bis[(2,4,6-triiodo-5-acétamido-3-amino-phényl)amino]propane

5g de 2,4,6-triiodo-5-acétamido-1,3-diamino benzène (Exemple 2e) sont ajoutés à la température ambiante sur une suspension de 0,5 g d'hydrure de sodium à 50 % dans 100 $cm^3$ de DMF anhydre.

Après ½ heure à 50 °C sous azote, 0,5 $cm^3$ de 1,3-dibromo propane sont ajoutés goutte à goutte. Le mélange réactionnel est laissé une nuit à 50 °C, concentré à sec puis repris à l'eau.

Après filtration, le produit est purifié par chromatographie sur colonne de silice (éluant éther-méthanol 95/5). 2,5 g de produit crème sont récupérés avec un rendement de 58 %.

- CCM Eluant 2 $R_f$ = 0,71
- Pureté en iode 98 %

$^1$H RMN (DMSO D6) ; s, 1,75 (6H $COCH_3$) ; m 2 (2H $C-CH_2-C$) ; m 3,1 à 3,7 (4H $CH_2-C-CH_2$) ; s 5,4 (8H $NH_2$).

b) préparation du 1,3-bis[N-(2,4,6-triiodo-3,5-acétamido-phényl)acétamido]propane

2 g (0,00178 moles) du composé obtenu en a) sont ajoutés sur une solution sous azote à 80 °C composée de 10 cm³ d'acide acétique anhydre et de 1,14 cm³ d'anhydride acétique. Après addition de 3 gouttes d'acide sulfurique à 35 %, le milieu réactionnel est laissé ½ heure à 80 °C, refroidi et hydrolysé par addition de 20 cm³ d'eau. Après filtration le produit est purifié par dissolution dans la soude, décoloration au noir et précipitation à pH 1. Rendement : 50 %

- Pureté en iode 97,5%
- CCM Eluant 2 $R_f$ = 0,24

$^1$H RMN (DMSO D6): m, 1,2 (2H $C-CH_2-C$) ; 2 s, 1,7 et 2 (18H-$COCH_3$) ; m 3,6 (4H $CH_2-C-CH_2$); m 10 (6H NH-CO)

c) préparation du 1,3-bis{N-(2,4,6-triiodo-3,5-bis[N-(2,3-dihydroxy propyl)acétamido]phényl)acétamido}propane

3 g du composé obtenu en b) sont dissous dans 24 cm³ de soude 1N.

2,8 cm³ de chloropropane-2,3-diol et 10 cm³ de soude 1N sont additionnés lentement et simultanément sur le milieu réactionnel à 70 °C.

Après 12 heures de réaction à 70 °C le mélange réactionnel est décoloré au noir, neutralisé à pH7 évaporé à sec puis repris au méthanol, les sels minéraux sont éliminés par filtration et le produit est purifié sur silice silanisée

par élution successive avec l'eau et le méthanol on récupère ainsi 1,2 g de produit avec un rendement de 33%

- pureté en iode 98,1%
- CCM éluant MeOH/Acétate d'éthyle/$NH_3$, $H_2O$ (28%) (15/30/10) Rf = 0,50-0,34

Les composés selon l'invention se sont avérés être stables à la stérilisation, c'est ainsi que les composés obtenus aux exemples 3, 8 et 9 traités à 121 °C pendant 10 mn ne présentent pas de modifications.

On donnera ci-après des résultats d'essais mettant en évidence l'intérêt des composés de l'invention.

1) Neurotoxicité

- la toxicité aiguë des composés a été déterminée sur souris femelles (poids 20 g) de souche SWISS $OF_1$ par administration intra-cérébrale selon la technique décrite par HOLEY et Mc CORMICK dans : Pharmacological effects produced by intracerebral injection of drugs in the conscious mouse BRIT.J.PHARMACOL. 12, 2. 1957.

5 animaux sont utilisés par dose, les volumes administrés sont de 0,05 $cm^3$ - 0,07 $cm^3$ - 0,10 $cm^3$ par souris soit 2,5 - 3,5 et 5 $cm^3$ par kg, avec des vitesses d'injection lentes.

La toxicité (DL50) est notée 7 jours après l'administration.

2) Osmolalité

- l'osmolalité exprimée en mosmoles par kg, est déterminée par cryoscopie à l'aide d'un osmomètre de type FISKE-OS Les résultats sont donnés dans le tableau suivant où l'on a également donné la solubilité des composés

| Composés Exemples | Solubilité en moles/litre | DL50 IC en mmoles par kg | Osmolalité mosmol par kg |
|---|---|---|---|
| 3 | 1 | 4 | 630 |
| 8 | 0,94 | 3,3 | |
| 9 | 1 | 3,5 | 690 |

 (tous pays sauf AT)

# REVENDICATIONS

1 - Composés choisis parmi les composés de formule

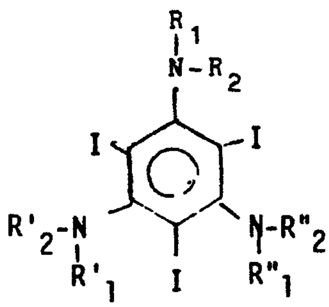

I

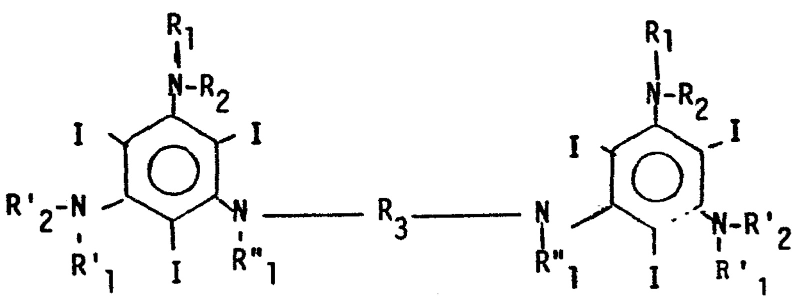

$II_a$

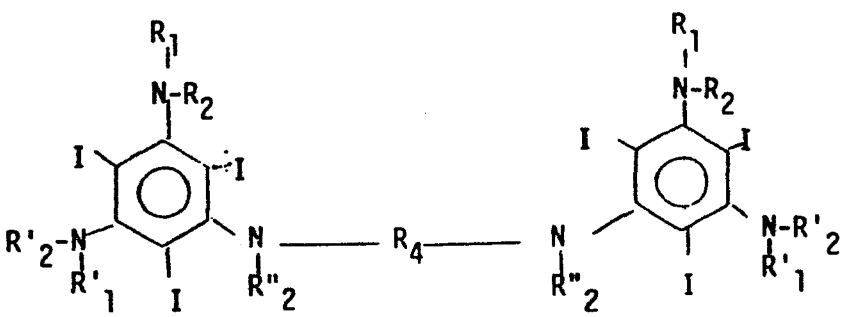

$II_b$

dans lesquelles

$R_1$, $R'_1$ et $R''_1$ sont choisis indépendamment les uns des autres parmi un atome d'hydrogène, un groupe alkyle en $C_1$ à $C_4$, un groupe hydroxyalkyle en $C_1$ à $C_4$, un groupe polyhydroxyalkyle en $C_1$ à $C_6$, un groupe alkoxy ($C_1$-$C_4$) hydroxyalkyle ($C_1$-$C_4$), et un groupe hydroxyalkoxy ($C_1$-$C_4$) alkyle ($C_1$-$C_4$),

$R_2$, $R'_2$ et $R''_2$ sont choisis indépendamment les uns des autres parmi les groupes acyle de formule

$$-\underset{\underset{O}{\|}}{C}-R_5$$

$R_5$ étant un groupe alkyle en $C_1$ à $C_4$, hydroxyalkyle en $C_1$ à $C_4$, polyhydroxyalkyle en $C_1$ à $C_6$ ou alkoxy ($C_1$-$C_4$) alkyle ($C_1$-$C_4$),

$R_3$ est un groupe choisi parmi les groupes diacyle de formule

$$- CO - R_6 - CO -$$

$R_6$ étant une simple liaison, un groupe alkylène en $C_1$ à $C_4$, un groupe hydroxyalkylène en $C_1$ à $C_4$, un groupe polyhydroxyalkylène en $C_1$ à $C_4$ ou un groupe alkoxy ($C_1$-$C_4$) alkylène ($C_1$-$C_4$)

$R_4$ est un groupe choisi parmi les groupes de formule

$$- CH_2 - R_6 - CH_2 -$$

$R_6$ ayant la signification donnée précédemment.

2 - Composés selon la revendication 1, de formule :

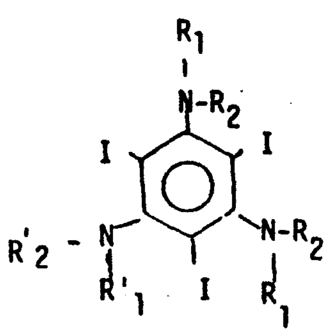

Ia

dans laquelle $R_1$, $R'_1$, $R_2$ et $R'_2$ ont la signification donnée à la revendication 1.

3. Composés selon la revendication 1, de formule

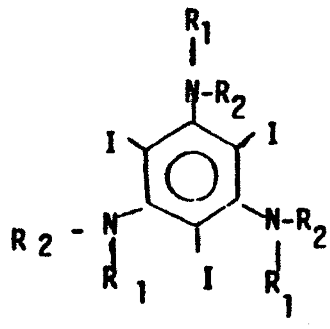

$I_a$,

dans laquelle $R_1$ et $R_2$ ont la signification donnée à la revendication 1.

4 - Composés selon la revendication 1 de formule I dans laquelle au moins l'un des substituants de chaque atome d'azote est un radical présentant au moins un groupe hydroxy ou alkoxy.

5 - Composés selon la revendication 4, dans lesquels $R_1$, $R'_1$ et $R''_1$ sont des groupes hydroxyalkyle en $C_1$ à $C_4$, polyhydroxy alkyle en $C_1$ à $C_6$ ou hydroxy alkoxy $(C_1-C_4)$ alkyl $(C_1-C_4)$.

6 - Composés selon la revendication 1 qui sont le 2,4,6-triiodo-1,3,5,-tris[N-(2,3-dihydroxy propyl)acétamido]benzène, le 2,4,6-triiodo-1-(méthoxyacétamido) - 3,5-bis [N-(2,3-dihydroxypropyl)acétamido]benzène , le 2,4,6-triiodo -1-(2-hydroxypropionyl-amino)-3,5-bis [N-(2,3-dihydroxy-propyl) acétamido] benzène et le 2,4,6-triiodo-1-acétamido-3,5-[N-(2,3 - dihydroxypropyl)acétamido] benzène.

7 - Produit de contraste caractérisé en ce qu'il contient au moins un composé selon l'une quelconque des revendications 1 à 6.

8 - Produit de contraste selon la revendication 7, caractérisé en ce qu'il est constitué par une solution aqueuse du ou des composés.

9 - Procédé de préparation d'un composé de formule I telle que définie à la revendication 1, caractérisé en ce que l'on effectue une acylation avec un réactif acylant $R_5$-CO-Z (Z étant un reste chlorure ou $-O-CO-R_5$) d'un composé choisi parmi les composés de formule

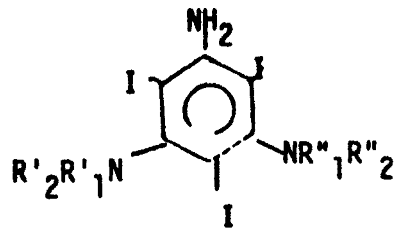

III

dans laquelle $R'_1$, $R''_1$, $R'_2$ et $R''_2$ ont les significations données précédemment ou sont des groupes correspondants dont les groupes hydroxy sont protégés,
et les composés de formule

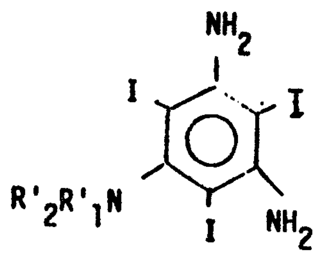

IV

dans laquelle $R'_1$ et $R'_2$ ont les significations données précédemment ou sont des groupes correspondants dont les groupes hydroxy sont protégés
donnant respectivement des composés de formule

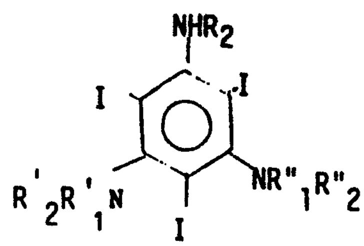

Ib

et

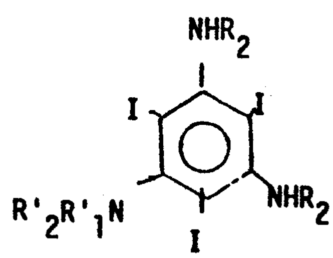

Ic

cette réaction étant éventuellement suivie d'une déprotection des groupes hydroxy et/ou d'une alkylation par un agent alkylant $R_1Z'$ ($R_1$ ayant la signification donnée précédemment mais autre que l'hydrogène et Z' étant un groupe labile tel qu'un halogène) de façon à obtenir un composé de formule I ou Ia.

10- Procédé selon la revendication 9, caractérisé en ce que les composés de formule III et IV sont obtenus à partir des benzamides correspondants de formule

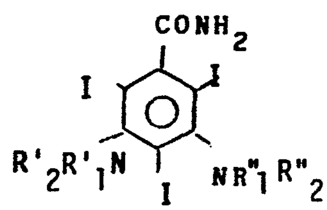

V

et

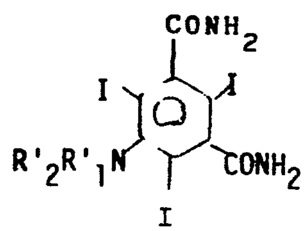

VI

par une réaction de réarrangement de type Hofmann.

## REVENDICATIONS

1 - Procédé de préparation d'un composé de formule

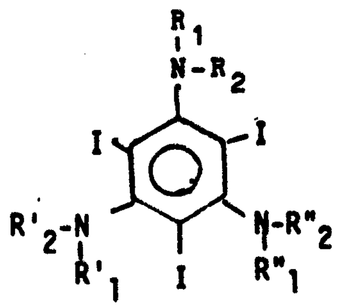

I

dans laquelle

$R_1$, $R'_1$ et $R"_1$ sont choisis indépendamment les uns des autres parmi un atome d'hydrogène, un groupe alkyle en $C_1$ à $C_4$, un groupe hydroxyalkyle en $C_1$ à $C_4$, un groupe polyhydroxyalkyle en $C_1$ à $C_6$, un groupe alkoxy $(C_1-C_4)$ hydroxyalkyle $(C_1-C_4)$, et un groupe hydroxyalkoxy $(C_1-C_4)$ alkyle $(C_1-C_4)$,

$R_2$, $R'_2$ et $R"_2$ sont choisis indépendamment les uns des autres parmi les groupes acyle de formule

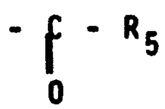

$R_5$ étant un groupe alkyle en $C_1$ à $C_4$, hydroxyalkyle en $C_1$ à $C_4$, polyhydroxyalkyle en $C_1$ à $C_6$ ou alkoxy $(C_1-C_4)$ alkyle $(C_1-C_4)$,

caractérisé en ce que l'on effectue une acylation avec un réactif acylant $R_5$-CO-Z (Z étant un reste chlorure ou -O-CO-$R_5$) d'un composé choisi parmi les composés de formule

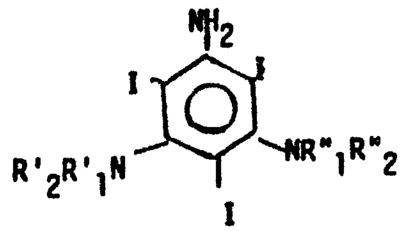

III

dans laquelle $R'_1$, $R''_1$, $R'_2$ er $R''_2$ ont les significations données précédemment ou sont des groupes correspondants dont les groupes hydroxy sont protégés,
et les composés de formule

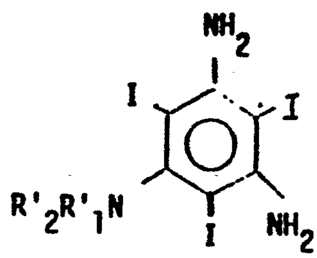

IV

dans laquelle $R'_1$ et $R'_2$ ont les significations données précédemment ou sont des groupes correspondants dont les groupes hydroxy sont protégés
donnant respectivement des composés de formule

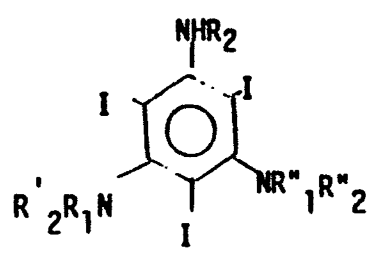

Ib

et

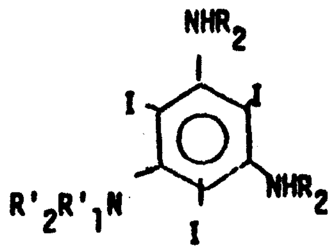

Ic

cette réaction étant éventuellement suivie d'une déprotection des groupes hydroxy et/ou d'une alkylation par un agent alkylant $R_1Z'$ ($R_1$ ayant la signification donnée précédemment mais autre que l'hydrogène et Z' étant un groupe labile.

2 - Procédé selon la revendication 1, caractérisé en ce que les composés de formule III et IV sont obtenus à partir des benzamides correspondants de formule

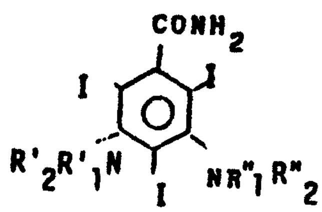

V

et

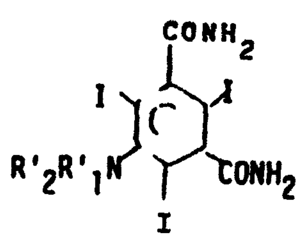

VI

par une réaction de réarrangement de type Hofmann.

3 - Procédé de préparation de composés de formule

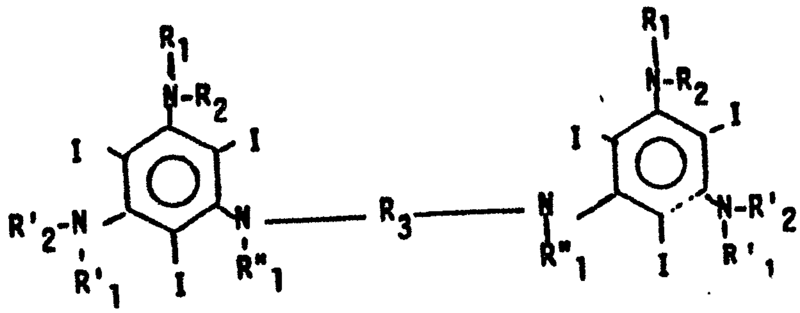

(II$_a$)

dans laquelle

$R_1$, $R'_1$ et $R''_1$ sont choisis indépendamment les uns des autres parmi un atome d'hydrogène, un groupe alkyle en $C_1$ à $C_4$, un groupe hydroxyalkyle en $C_1$ à $C_4$, un groupe polyhydroxyalkyle en $C_1$ à $C_6$, un groupe alkoxy ($C_1$-$C_4$) hydroxyalkyle ($C_1$-$C_4$), et un groupe hydroxyalkoxy ($C_1$-$C_4$) alkyle ($C_1$-$C_4$),

$R_2$ et $R'_2$ sont choisis indépendamment les uns des autres parmi les groupes acyle de formule

$$-\underset{\underset{O}{\|}}{C}-R_5$$

$R_5$ étant un groupe alkyle en $C_1$ à $C_4$, hydroxyalkyle en $C_1$ à $C_4$, polyhydroxyalkyle en $C_1$ à $C_6$ ou alkoxy ($C_1$-$C_4$) alkyle ($C_1$-$C_4$),

$R_3$ est un groupe choisi parmi les groupes diacyle de formule

$$-CO-R_6-CO-$$

$R_6$ étant une simple liaison, un groupe alkylène en $C_1$ à $C_4$, un groupe hydroxyalkylène en $C_1$ à $C_4$, un groupe polyhydroxyalkylène en $C_1$ à $C_4$ ou un groupe alkoxy ($C_1$-$C_4$) alkylène ($C_1$-$C_4$),

caractérisé en ce que l'on effectue une réaction d'un composé de formule

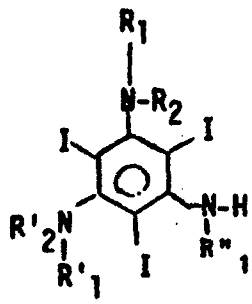

(XXI)

dans laquelle $R_1$, $R'_1$, $R''_1$, $R_2$ et $R'_2$ ont la signification donnée ci-dessus avec un agent biacylant de formule

$Cl - CO - R_6 - COCl$ (XXII)

4 - Procédé de préparation de composés de formule

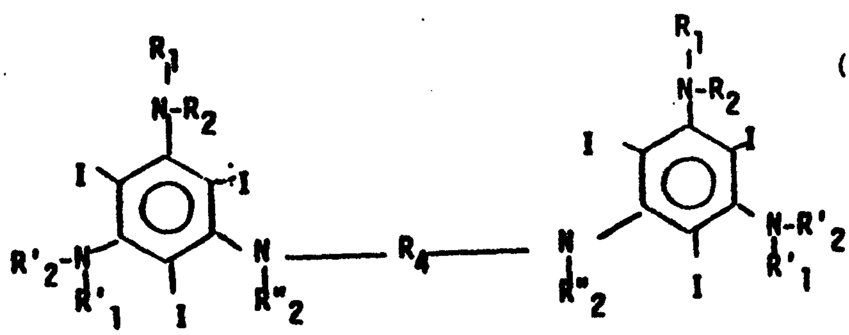

($II_b$)

dans laquelle

$R_1$ et $R'_1$ sont choisis indépendamment les uns des autres parmi un atome d'hydrogène, un groupe alkyle en $C_1$ à $C_4$, un groupe hydroxyalkyle en $C_1$ à $C_4$, un groupe polyhydroxyalkyle en $C_1$ à $C_6$, un groupe alkoxy ($C_1$-$C_4$) hydroxyalkyle ($C_1$-$C_4$), et un groupe hydroxyalkoxy ($C_1$-$C_4$) alkyle ($C_1$-$C_4$),

$R_2$, $R'_2$ et $R''_2$ sont choisis indépendamment les uns des autres parmi les groupes acyle de formule

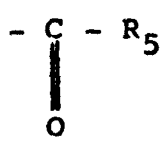

$R_5$ étant un groupe alkyle en $C_1$ à $C_4$, hydroxyalkyle en $C_1$ à $C_4$, polyhydroxyalkyle en $C_1$ à $C_6$ ou alkoxy ($C_1$-$C_4$) alkyle ($C_1$-$C_4$),

$R_4$ est un groupe choisi parmi les groupes de formule

$- CH_2 - R_6 - CH_2 -$,

$R_6$ étant une simple liaison, un groupe alkylène en $C_1$ à $C_4$, un groupe hydroxyalkylène en $C_1$ à $C_4$, un groupe polyhydroxyalkylène en $C_1$ à $C_4$ ou un groupe alkoxy ($C_1$-$C_4$) alkylène ($C_1$-$C_4$), caractérisé en ce que l'on effectue une réaction d'un composé de formule

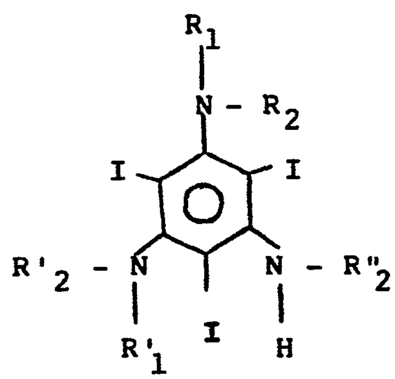

avec un agent bialkylant de formule

$X - R_4 - X$

dans laquelle X = Cl, Br ou I.

5 - Procédé de préparation d'un produit opacifiant caractérisé en ce que l'on met sous une forme administrable un composé choisi parmi les composés de formule

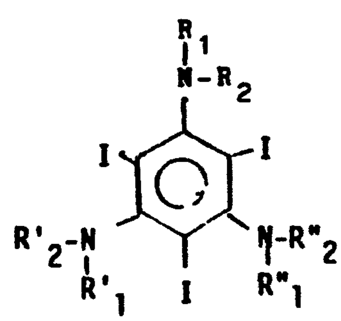

(I)

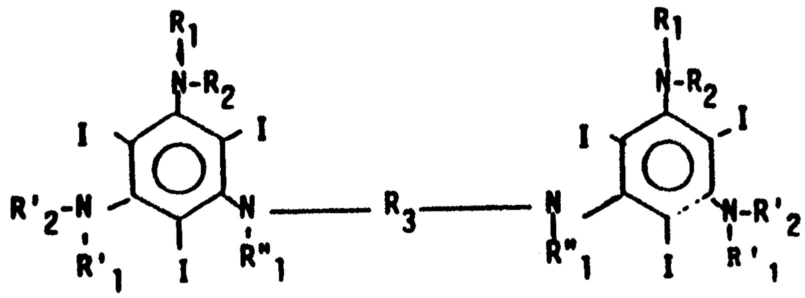

$(II_a)$

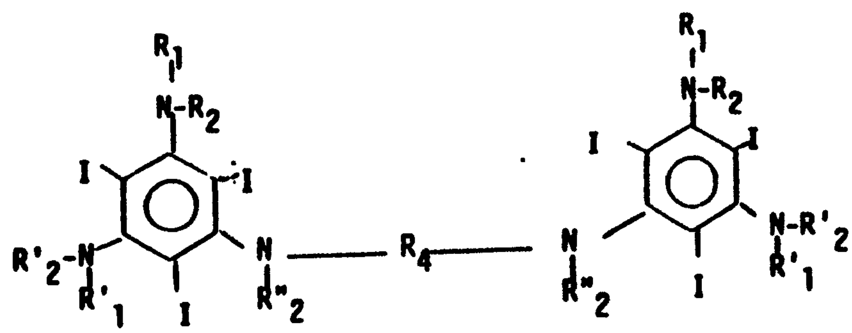

$(II_b)$

dans lesquelles

$R_1$, $R'_1$ et $R''_1$ sont choisis indépendamment les uns des autres parmi un atome d'hydrogène, un groupe alkyle en $C_1$ à $C_4$, un groupe hydroxyalkyle en $C_1$ à $C_4$, un groupe polyhydroxyalkyle en $C_1$ à $C_6$, un groupe alkoxy $(C_1-C_4)$ hydroxyalkyle $(C_1-C_4)$, et un groupe hydroxyalkoxy $(C_1-C_4)$ alkyle $(C_1-C_4)$,

$R_2$, $R'_2$ et $R''_2$ sont choisis indépendamment les uns des autres parmi les groupes acyle de formule

$$-\underset{\underset{O}{\|}}{C}-R_5$$

$R_5$ étant un groupe alkyle en $C_1$ à $C_4$, hydroxyalkyle en $C_1$ à $C_4$, polyhydroxyalkyle en $C_1$ à $C_6$ ou alkoxy $(C_1-C_4)$ alkyle $(C_1-C_4)$.

$R_3$ est un groupe choisi parmi les groupes diacyle de formule

$$- CO - R_6 - CO -$$

$R_6$ étant une simple liaison, un groupe alkylène en $C_1$ à $C_4$, un groupe hydroxyalkylène en $C_1$ à $C_4$, un groupe polyhydroxyalkylène en $C_1$ à $C_4$ ou un groupe alkoxy ($C_1$-$C_4$) alkylène ($C_1$-$C_4$)

$R_4$ est un groupe choisi parmi les groupes de formule

$$- CH_2 - R_6 - CH_2 -$$

$R_6$ ayant la signification donnée précédemment.

**0177414**

Office européen des brevets

# RAPPORT DE RECHERCHE EUROPEENNE

Numéro de la demande

EP 85 40 1878

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | FR-A-2 313 917 (CORTIAL)<br>* Exemples; revendications *<br>----- | 1-5 | A 61 K 49/04<br>C 07 C 103/44<br>C 07 C 103/49 //<br>C 07 C 103/28<br>C 07 C 103/24 |
| | | | **DOMAINES TECHNIQUES RECHERCHES (Int. Cl.4)** |
| | | | A 61 K 49/00<br>C 07 C 103/00 |

Le présent rapport de recherche a été établi pour toutes les revendications

| Lieu de la recherche | Date d'achèvement de la recherche | Examinateur |
|---|---|---|
| LA HAYE | 08-01-1986 | PAUWELS G.R.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant